# EUROPEAN PATENT APPLICATION

(11) **EP 1 767 245 A1**
(43) Date of publication of application: **28.03.2007**
(21) Application number: 05380235.1
(22) Date of filing: 24.10.2005
(51) Int. Cl.: A61N 1/39

(54) **External defibrillation device for cardiac resuscitation**

(30) Priority: 21.09.2005 ES 200502070 U
(71) Applicant: Cardioprotection, S.L., 28023 Madrid (ES)
(72) Inventor: Menendez Fernandez, Justo, 28220 Majadahonda (Madrid) (ES)
(74) Representative: Gonzalez Palmero, Fé

(57) **Abstract**

External defibrillation device for cardiac resuscitation, which is specially designed for episodes of ventricular fibrillation, and more specifically for resuscitation treatment by means of electrical impulses, characterised in that the automated external defibrillator, which can be of any conventional type, is housed in a column that may be installed either outside or inside the hospital environment, particularly in public places where there are a lot of people, with the defibrillator housed in the column and accessible via a lockable door, to enable said defibrillator to be used immediately by any authorised person regardless of whether this person is a member of the medical staff.

## Description

### OBJECT OF THE INVENTION

The present invention relates to a device for cardiac resuscitation and defibrillation that has been specially designed to achieve resuscitation or cardiac recovery in a situation of sudden cardiac death due to ventricular fibrillation.

The object of the invention is to make it possible to have an automated external defibrillator anywhere that is considered appropriate, not only in hospitals, which can be used without the need for the participation of professional medical staff in order to enable a rapid response in an emergency situation, being simple to use, with instructions on the defibrillator.

### BACKGROUND OF THE INVENTION

As is well known, the primary cause of death in developed countries is cardiovascular diseases, and sudden cardiac death is one of the most important of these.

Most sudden cardiac deaths are due to ventricular fibrillation, a cardiac arrhythmia where chaotic electrical activity prevents the mechanical activity of the heart. Without treatment it inevitably leads to death within minutes. The only effective treatment for ventricular fibrillation is electrical defibrillation, and the sooner it is applied, the greater its chances of being effective.

Up until now, electrical defibrillator apparatus have formed part of the equipment of a hospital, a specialised mobile unit, an ambulance or similar places, so that when a case of sudden cardiac death occurs, which usually happens outside a hospital, it is necessary to call the emergency services and wait for the arrival of a vehicle that is suitably equipped to treat the patient, which in most cases means wasting so much time that the patient has already died when the treatment arrives.

In fact, 80% of cases of sudden cardiac death occur outside the hospital environment, and in 97% of cases the patients die as a result of the absence or failure of resuscitation treatment.

### BACKGROUND OF THE INVENTION

The cardiac resuscitation and automated external defibrillation device proposed by this invention represents a considerable technological advance in solving said problem, so that said defibrillator, with a number of accessories which will be mentioned below, can be installed inside or outside hospitals, in any places where its use could be considered necessary, preferably in public places with a large number of people, in order to allow its rapid availability and its use by people who are not healthcare specialists, as the use of the automated external defibrillator is very simple and intuitive, and the accessories of said defibrillator include panels with information about how to use it.

This means that in a case of sudden cardiac death the patient can receive resuscitation treatment, by electrical defibrillation, almost immediately and with a high probability that the resuscitation will be effective.

More specifically, the invention takes the form of a type of column or tower unit that can be free-standing, or hung from a wall or from the ceiling, according to the space available in each case. The column houses the defibrillator, which can be of any appropriate conventional type, and it has an access door with a lock to prevent tampering by unauthorised persons.

The aforementioned lock should preferably be an electronic lock, controlled by an I.D. card, alphanumeric keypad, etc.

The defibrillator is fed by a power supply connected to the mains network, although it should preferably have an auxiliary battery to allow it to work on its own in the event of a power cut.

A control circuit or central processing unit controls the aforementioned parts, and is also connected, by means of an alarm switch, to an alarm system that is duly positioned in the proximity of the column, so that the person or people in charge of using it receive the corresponding emergency signal, whilst the column is also linked, by means of a telephone module, modem, or any other suitable transmission system, to a control centre that can receive information and send orders to a suitably distributed battery or set of defibrillators.

### DESCRIPTION OF THE DRAWINGS

To complement this description and in order to aid a better understanding of the invention's characteristics, according to a preferred embodiment thereof, a set of illustrative and non-limiting drawings integral to said description is attached, which are as follows:
Figure 1.- Shows, according to a general perspective view, an embodiment of a defibrillator for cardiac resuscitation according to the object of the present invention.
Figure 2.- Shows a diagram of the defibrillator referred to in the previous figure.

### PREFERRED EMBODIMENT OF THE INVENTION

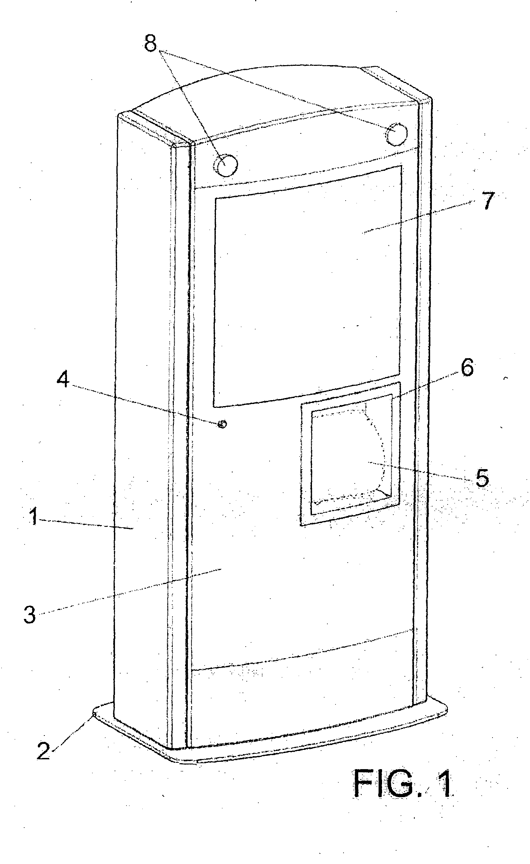

In the embodiment chosen in figure 1, the defibrillator takes the form of a column (1), which is free-standing thanks to a base or foot (2) that gives it sufficient stability, having a door (3) on the front, with its corresponding lock (4), which provides access to the defibrillator (5), which is housed inside the column (1) and is directly visible through the transparent window (6), also having on said door (3) an information panel (7), and at the top there are pilot lights (8) corresponding to an alarm system that is activated by a switch (9).

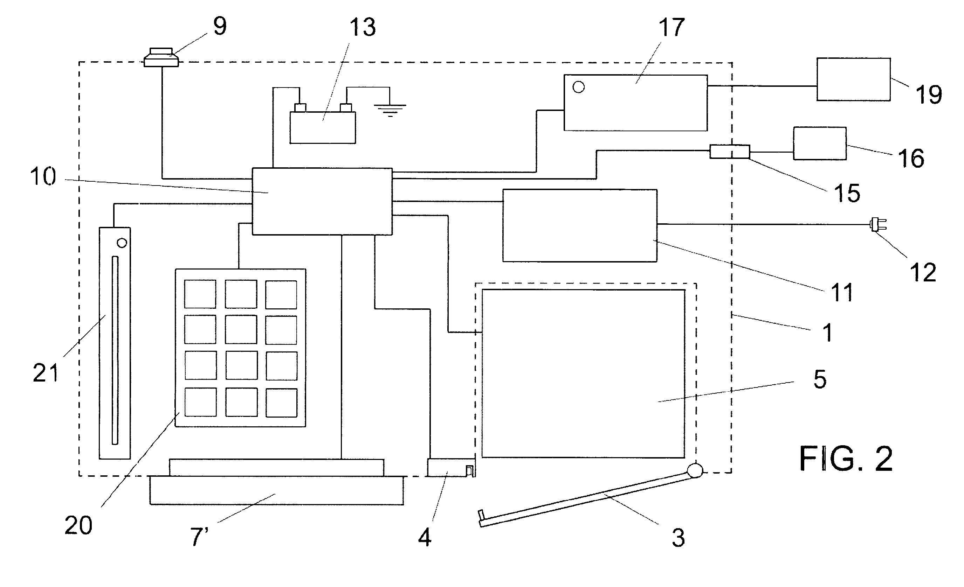

More specifically, and as shown by the diagram in figure 2, the defibrillator (5) is assisted by a central processing unit (10), through which said defibrillator (5) is fed by a power supply (11) which duly converts the mains voltage that reaches the equipment via the plug (12), although the defibrillator also has an auxiliary battery (13) that gives it sufficient autonomy in the event of a power cut.

This control circuit (10) also supplies two pilot lights (8) on the front of the column, whilst simultaneously sending said alarm signal, via a connector (14), to an alarm system (16) that has been duly established in the building wherein the column (1) is installed, so that the person or people in charge of using the defibrillator, can be alerted as rapidly as possible of the existence of an emergency situation and go to the scene of the incident.

The central processing unit (10) also sends information, via a telephone module or modem (17), and more specifically via the telephone line (18) to a control centre (19).

The instructions for using the equipment can be printed on the panel (7) of the door, but it preferably has a configurable information panel (7'), which is also controlled by the central processing unit (10).

The lock (4) can be any conventional mechanical lock, but preferably it should be possible to operate it using said central unit (10) by means of an alphanumeric keypad (20) into which the corresponding code is entered, or by means of a card reader (21) that allows the person to be identified using the corresponding card, in order to open the door (3).

## Claims

1. External defibrillation device for cardiac resuscitation, which is specially designed for episodes of ventricular fibrillation, and more specifically for resuscitation treatment by means of electrical impulses, **characterised in that** the automated external defibrillator, which can be of any conventional type, is housed in a column that may be installed either outside or inside the hospital environment, particularly in public places where there are a lot of people, with the defibrillator housed in the column and accessible via a lockable door, to enable said defibrillator to be used immediately by any authorised person regardless of whether this person is a member of the medical staff.

2. External defibrillation device for cardiac resuscitation, according to claim 1, **characterised in that** the defibrillator is fed by a power supply connected to the mains network, having an auxiliary battery inside the column to supply the defibrillator in the event of a power cut.

3. External defibrillation device for cardiac resuscitation, according to the previous claims, **characterised in that** the lock to access the inside of the column can be operated by a key, an alphanumeric keypad or a card reader, or by any other means that prevent access to the defibrillator by unauthorised persons.

4. External defibrillation device for cardiac resuscitation, according to the previous claims, **characterised in that** it includes alarm signals that can be activated using an alarm switch, either on the column or in any pre-established place in the building wherein said column is installed.

5. External defibrillation device for cardiac resuscitation, according to the previous claims, **characterised in that** it is connected to a control centre via a telephone module or modem and the telephone line.

6. External defibrillation device for cardiac resuscitation, according to the previous claims, **characterised in that** it includes an information panel with instructions about how to use the defibrillator.
